# EUROPEAN PATENT SPECIFICATION

(11) **EP 4 175 492 B1**
(45) Date of publication and mention of the grant of the patent: **31.12.2025**
(21) Application number: 21810444.6
(22) Date of filing: 11.10.2021
(51) Int. Cl.: A23L 33/105, A23D 9/007, A23K 20/111, A23K 20/158, A23L 33/115, A61K 8/92, A61K 36/63, A61K 47/44, C11B 1/00, C11B 1/06, A61Q 19/00, C11B 1/04

(54) **INTEGRATED PROCESS FOR THE ECO-SUSTAINABLE PRODUCTION OF HIGH QUALITY EXTRA VIRGIN OLIVE OIL AND MICRONIZED BY PRODUCTS**
INTEGRIERTES VERFAHREN ZUR ÖKOLOGISCH NACHHALTIGEN HERSTELLUNG VON HOCHWERTIGEM EXTRANÄREM OLIVENÖL UND MIKRONISIERTEN NEBENPRODUKTEN
PROCESSUS INTÉGRÉ DE PRODUCTION ÉCOLO-DURABLE D'HUILE D'OLIVE VIERGE EXTRA DE HAUTE QUALITÉ ET DE SOUS-PRODUITS MICRONISÉS

(30) Priority: 09.10.2020 IT 202000023827
(43) Date of publication of application: 10.05.2023
(73) Proprietor: Manni Oil Società Agricola S.r.l., 00154 Roma (IT)
(72) Inventor: MANNI, Armando, 00154 Roma (IT); ROMANI, Annalisa, 00154 Rome (IT); URCIUOLI, Silvia, 00154 Roma (IT); VITA, Chiara, 00154 Roma (IT)
(74) Representative: Banchetti, Marina
(86) International application number: PCT/IT2021/050326
(87) International publication number: WO 2022/074699

(56) References cited:
- US-A1- 2020 046 791
- NAZIRI ELENI ET AL: "Valorization of the major agrifood industrial by-products and waste from Central Macedonia (Greece) for the recovery of compounds for food applications", FOOD RESEARCH INTERNATIONAL, vol. 65, 1 November 2014 (2014-11-01), AMSTERDAM, NL, pages 350 - 358, XP055888052, ISSN: 0963-9969, DOI: 10.1016/j.foodres.2014.09.013
- DATABASE GNPD [online] MINTEL; 11 May 2018 (2018-05-11), ANONYMOUS: "Fruity Extra Virgin Olive Oil", XP055814875, retrieved from https://www.gnpd.com/sinatra/recordpage/5670381/ Database accession no. 5670381
- PANTZIAROS ALEXIS G ET AL: "A new olive oil production scheme with almost zero wastes", BIOMASS CONVERSION AND BIOREFINERY, vol. 11, no. 2, 8 February 2020 (2020-02-08), pages 547 - 557, XP037398642, ISSN: 2190-6815, DOI: 10.1007/S13399-020-00625-0
- ROMERO-GARCÍA J M ET AL: "Biorefinery based on olive biomass. State of the art and future trends", BIORESOURCE TECHNOLOGY, ELSEVIER, AMSTERDAM, NL, vol. 159, 25 March 2014 (2014-03-25), pages 421 - 432, XP028646324, ISSN: 0960-8524, DOI: 10.1016/J.BIORTECH.2014.03.062
- ORDOUDI STELLA ET AL: "The Potential of Tree Fruit Stone and Seed Wastes in Greece as Sources of Bioactive Ingredients", RECYCLING, vol. 3, no. 1, 5 March 2018 (2018-03-05), pages 9, XP055888126, DOI: 10.3390/recycling3010009
- MARTÍN-GARCÍA BEATRIZ ET AL: "Box-Behnken experimental design for a green extraction method of phenolic compounds from olive leaves", INDUSTRIAL CROPS AND PRODUCTS, ELSEVIER, NL, vol. 154, 4 July 2020 (2020-07-04), XP086225179, ISSN: 0926-6690, [retrieved on 20200704], DOI: 10.1016/J.INDCROP.2020.112741
- HOSSAIN MD ARAFAT ET AL: "Fluorescence-Based Determination of Olive Oil Quality Using an Endoscopic Smart Mobile Spectrofluorimeter", IEEE SENSORS JOURNAL, IEEE SERVICE CENTER, NEW YORK, NY, US, vol. 20, no. 8, 23 December 2019 (2019-12-23), pages 4156 - 4163, XP011779194, ISSN: 1530-437X, [retrieved on 20200317], DOI: 10.1109/JSEN.2019.2961419
- DATABASE GNPD [online] MINTEL; 11 May 2018 (2018-05-11), ANONYMOUS: "Fruity Extra Virgin Olive Oil", XP055814875, retrieved from https://www.gnpd.com/sinatra/recordpage/5670381/ Database accession no. 5670381
- AHMAD-QASEM MARGARITA H ET AL: "Drying and storage of olive leaf extracts. Influence on polyphenols stability", INDUSTRIAL CROPS AND PRODUCTS, ELSEVIER, NL, vol. 79, 19 November 2015 (2015-11-19), pages 232 - 239, XP029362406, ISSN: 0926-6690, DOI: 10.1016/J.INDCROP.2015.11.006
- DOGU-BAYKUT ESRA ET AL: "Ultraviolet (UV-C) radiation as a practical alternative to decontaminate thyme ( Thymus vulgaris L.)", JOURNAL OF FOOD PROCESSING AND PRESERVATION, vol. 43, no. 6, 16 November 2018 (2018-11-16), TRUMBULL, CT, US, pages e13842, XP055888078, ISSN: 0145-8892, Retrieved from the Internet <URL:https://onlinelibrary.wiley.com/doi/full-xml/10.1111/jfpp.13842> DOI: 10.1111/jfpp.13842
- GULLÓN PATRICIA ET AL: "Valorization of by-products from olive oil industry and added-value applications for innovative functional foods", FOOD RESEARCH INTERNATIONAL, ELSEVIER, AMSTERDAM, NL, vol. 137, 18 September 2020 (2020-09-18), XP086360345, ISSN: 0963-9969, [retrieved on 20200918], DOI: 10.1016/J.FOODRES.2020.109683

## Description

### Field of the invention

The present invention relates to an integrated process for the eco-sustainable production of high-quality extra virgin olive oil and micronized by-products rich in polyphenols, according to the principles of circular economy. More specifically, the invention relates to a multifunctional process which, starting from *Olea europaea,* produces an extra virgin olive oil (EVO) with a high content of hydroxytyrosol and its derivatives, and in which the stoned and de-oiled olive pomace exiting the mill is directly transformed into a powder product useful in the field of food and nutraceutics, with a high content of antioxidant active principles. Also the leaves and apical twigs resulting from the olive harvest are directly treated in the same plant, to obtain a powder product standardized in the content of oleuropein (the bitter active ingredient of the olive fruit), which is a cardioprotective molecule having an antioxidant character.

### Background of the invention

Extra virgin olive oil (EVO) is a fundamental part of the gastronomic culture and Mediterranean diet, and it is known that its content in fatty acids, tocopherols and minor polar compounds (antioxidants) plays an important role in the prevention of diseases of the cardiovascular system.

The minor polar compounds present in EVO oil can be grouped into at least four subclasses: simple phenols (tyrosol, hydroxytyrosol and derivatives), secoiridoids (oleuropein aglycone, 10-hydroxyoleocanthal, oleocanthal and secoiridoid derivatives), lignans (acetoxypinoresinol and pinoresinol) and flavonoid aglycones (mostly represented by luteolin and apigenin).

EVO oil is the only vegetable oil that naturally contains appreciable quantities of polyphenolic substances. These molecules, from a technological point of view, are largely responsible for the stability of the EVO oil, to which they give greater resistance to both self-oxidation and thermo-oxidation, also helping to increase the stability of the product.

The content of polyphenolic compounds in olive oil depends on various factors, including the cultivar, the degree of ripeness of the olives, the possible infestation by the olive fly *(Bactrocera oleae),* the climate, the times and the ways of storage, methods of pressing and squeezing. It has also been found that the qualitative and quantitative variations of polyphenols in oil lead to substantial changes at the organoleptic level.

The greater commercial awareness by traditional consumers of oil and the increasing consumption of EVO oil even in industrialized countries that are not traditionally consumers of this product has led to the enhancement and significance in the judgment of appreciation and in the choice of an extra virgin olive oil, the sensory peculiarities, especially of flavour and aroma, and the high nutritional characteristics. The latter are related to the content of antioxidants, such as phenolic compounds and tocopherols.

There are many beneficial properties ascribed to the phenolic composition of EVO oil, in particular the antimicrobial, antioxidant and beneficial effects of oleuropein and hydroxytyrosol have been documented (Benavente et al., "Antioxidant activity of phenolics extracted from Olea europaea L. leaves", Food Chemistry 2000; 68: 457). Hydroxytyrosol is considered the main active biophenol and the most powerful antioxidant, due to the presence of the catechol group in the molecule, while oleuropein is known for its antiatherogenic and cardioprotective activities (inhibition of LDL oxidation and platelet aggregation), hypoglycaemic, antihypertensive (vasodilator), antiviral (also against the HIV virus), anti-inflammatory, cytostatic (against McCoy cells), molluscicide, endocrine and enzymatic modulator.

In particular, the presence of hydroxytyrosol and its derivatives, and high levels of secoiridoids, increases the antioxidant activity of EVO oil, suggesting that oils rich in these compounds can provide greater protection to human health, as this activity can have beneficial effects. in accordance with the oxidative hypothesis of atherosclerosis (Franconi et al., "The Antioxidant Effect of Two Virgin Olive Oils Depends on the Concentration and Composition of Minor Polar Compounds", J Agric. Food Chem 2006; 54: 3121).

In a review of 2019 (Romani A. et al., "Health Effects of Phenolic Compounds Found in Extra-Virgin Olive Oil, By-Products, and Leaf of Olea europaea L.", Nutrients 2019, 11, 1776) all the most recent contributions from the scientific community were collected regarding the health effects of phenolic compounds present in extra virgin olive oil, by-products and olive leaves. As for EVO oil, the already known antioxidant and anti-inflammatory effects are joined by anticancer effects, pro-apoptotic effects in leukaemia cells and the modulation of the enterocyte response to oxidative and inflammatory stimuli. It should be noted that many beneficial effects on health are also attributable to olive leaf extracts, including cardioprotective, anticancer and adjuvant effects of chemotherapy therapies, reduction of melanoma cell vitality and anti-angiogenic activity in medium cultured with fibroblasts of senescence-associated secretory phenotype. As regards the biological activity of the by-products of *Olea europaea* L., the antibacterial action against *S. aureus, B. subtilis, E. coli* and P. *aeruginosa* is reported, as well as anti-proliferative and anti-inflammatory effects.

As prescribed by Regulation (EU) No. 432/2012, relating to the compilation of a list of health claims permitted on food products (on which the European Food Safety Authority, EFSA, plays the role of competent agency), the following is an acceptable indication for EVO oil:
*"Olive oil polyphenols contribute to the protection of blood lipids from oxidative stress."*

The conditions of use of the claim are expressed as follows: *"The claim may be used only for olive oil which contains at least 5 mg of hydroxytyrosol and its derivatives (e.g., oleuropein complex and tyrosol) per 20 g of olive oil. In order to bear the claim information shall be given to the consumer that the beneficial effect is obtained with a daily intake of 20 g of olive oil."* In the light of the foregoing, the possibility of having means to efficiently manage and control the olive oil extraction plant (oil mill), implementing innovative techniques and devices aimed at limiting the possibility of errors in the production process, and to obtain products of high and controlled quality.

It is also known that EVO oil is subjected to degradative phenomena during storage that reduce its quality level over time and cause the product to gradually lose its beneficial characteristics. For this reason, extra virgin olive oils have a recommended shelf life of no more than 18 months after bottling.

Considering the secondary products and waste materials of the olive oil supply chain, consisting mainly of oil extraction residues, such as pomace and olive mill waste water, but also in other plant tissues such as leaves, in recent years scientific evidence has led to an increase in demand for functional foods and beverages enriched in polyphenols, so that such plant materials are increasingly in demand. Moreover, their recovery and reuse from waste complies with the circular economy strategy, which has introduced the concept of "zero waste".

Taking into account that the recovery process is convenient from an economic and environmental point of view only if the final products are standardized and obtained using industrial and sustainable technologies, it is interesting to enhance the by-products and waste of the olive oil sector, through the optimization process and the study of new environmentally friendly technologies, in order to obtain energy and new products with high added value.

Two different types of extraction systems are currently used for the production of olive oil: the traditional oil mill, which implements a discontinuous process by pressing, and the continuous system, which operates by centrifugation. Both types of plant include a first processing phase in which the olives are first sorted to remove the leaves, then washed with water and subsequently reduced to a paste in a mechanical crusher (pan-mill in traditional mills, crushers with hammers, discs, rollers, etc. in continuous systems).

In the case of traditional mills, the crushed olive paste is loaded between the plates of hydraulic presses in special containers made of intertwined fibres and subjected to mechanical extraction of the oil, with the production of a solid residue, the pomace with crushed pits, and a liquid product, called "must". An oily fraction is then separated from the must by centrifugation, which constitutes the oil from the first pressing, while the aqueous fraction, called vegetable water, constitutes the processing waste.

In the continuous mills, the crushed olive paste undergoes a phase called malaxation, which serves to stir it in order to favour the aggregation of the micro-drops of oil, which are separated from the water and solid parts by mechanical means. The malaxation is followed by the mechanical extraction of the oil, which can be obtained by the action of centrifugal force.

In the case of continuous systems of the so-called "three-phase" type, the olive paste is treated for pressing in horizontal axis centrifugal extractors called "decanters", after having been suitably diluted with hot water. Three phases are obtained from the operation: first press olive oil, pomace and olive mill waste water. It is evident that in this case the aqueous fraction obtained as a residue is, based on the same number of olives processed, quantitatively higher than that obtained from extraction in a traditional mill, and with a lower content of dissolved solids. The need to improve the quality of the oil and to reduce the environmental impact of waste has prompted to consider the opportunity to modify the three-phase centrifugal extraction cycle by reducing the addition of process water. For this reason, the so-called "two-phase" extraction systems by centrifugation have been developed, in which no water is added to the decanter, and the by-product obtained consists of a single phase, consisting of a mixture of pomace and vegetation waters, the so-called wet pomace.

In current production systems, the olive pomace constitutes an expensive by-product to manage, which is not further treated in the mill but is normally delivered by the mills to the so-called pomace oil extracting plant (plant for the treatment of olive residues). In pomace oil extracting plants, pomace oil is obtained by extracting the residual amount of oil contained in the virgin pomace by using chemical extraction methods. Although initially it contains a very high percentage of polyphenols originally contained in olives, pomace oil has decidedly inferior qualitative and organoleptic characteristics to olive oil, above all because the oil left in the pomace deteriorates over time, and it is not processed immediately after the extraction of the EVO oil by mechanical means but must be transported to the pomace oil extracting plant. For these reasons, the pomace oil market is constantly declining, and this pushes operators in the sector, and especially small olive companies, to get rid of this by-product by dumping it illegally into the environment.

Pantziaros A. G. et al. ("A new olive oil production scheme with almost zero wastes", Biomass Conv. Bioref. 2021, 11: 547-557) discloses a process for EVO oil production operating on stoned olive paste, the olive stones being crushed and separated into a woody residue to be used as a bio-fuel, and a pomace paste without stone, from which pomace oil can be extracted with hexane.

Romero-Garcia J. M. et al. authored a review ("Biorefinery based on olive biomass. State of the art and future trends", Bioresource Technology 2014, 159: 421-432) which collects several methods to operate on by-products and residues of olive oil production.

US 2020/046791 A1 discloses a method of obtaining olive oil and at least one extract concentrated in polyphenols based on the previous removal of the whole stone, electric pulsing of the olive paste, dehydration under vacuum of the pulsed paste and subsequent separation of the oil by centrifugation. The degreased olive paste is dehydrated and trated by supercritical extraction to obtain olive extracts and olive flour.

Ordoudi S. A. et al. ("The Potential of Tree Fruit Stone and Seed Wastes in Greece as Sources of Bioactive Ingredients", Recycling 2018, 3: 9) reviewed the potential uses of the inedible parts of the tree fuits, wih specific focus on seeds, including olive pits, which are normally used to extract pomace oil.

Martin-Garcia B. et al. ("Box-Behnken experimental design for a green extraction method of phenolic compounds from olive leaves", Industrial Crops & Products 2020, 154: 112741) discloses the extraction of polyphenols from dried olive leaves, the extracts comprising more than 20% by weight of oleuropein.

Hossain A. et al. ("Fluorescence-Based Determination of Olive Oil Quality Using an Endoscopic Smart Mobile Spectrofluorimeter", IEEE Sensors J. 2019, 20(8): 4156-4153) discloses the use of optical sensors to evaluate the quality of olive oils.

The database GNPD [Online] Mintel ("Fruity Extra Virgin Olive Oil", 11 May 2018) discloses an example of EVO oil having at least 5 mg hydroxytyrosol per 20 g unrefined oil.

Ahmad-Qasem M. et al. ("Drying and storage of olive leaf extracts. Influence of polyphenols stability", Industrial Crops & Products 2015, 79: 232-239) discloses the drying of olive leaves in hot air or freeze drying, and the ethanolic extraction of the dried leaves.

Dogu-Baykut E. et al. ("Ultraviolet (UV-C) radiation as a practical alternative to decontaminate thyme (Thymus vulgaris L.)", J. Food Proc. Preserv. 2018, 43(6): e13842) discloses the use of UV-C rays to decontaminate herbs and spices.

In the light of the foregoing, the present invention, therefore, aims at developing an integrated process for the eco-sustainable production of high-quality extra virgin olive oil, effective for the protection of blood lipids from oxidative stress, and of traceable by-products with a high content of antioxidant active ingredients from virgin olive pomace and olive leaf, which also achieves the goal of eliminating waste from the olive-oil supply chain.

### Summary of the invention

For this purpose, according to the present invention, an integrated multifunctional process has been developed capable of achieving, with a non-destructive method, traceability and monitoring of the quality and degree of ripeness of the olives, the production of a traceable high-quality extra virgin olive oil, the production of freeze-dried and micronized products from stoned and de-oiled olive paste and olive leaf, for food, nutraceutical, cosmetic use, or even for animal feed or green, including biodynamic, agriculture, as well as for the production of vegetable water with a natural composition, reusable both in the washing processes of the raw material and in fertigation.

The proposed process aims to apply the principles of the circular economy, pursuing the objective of "zero waste" and generating, in the various production or treatment phases in the field, not waste but secondary raw materials traced in the content of principles active, coming from leaves and from stoned virgin olive pomace. These products are marketable, in a micronized form, standardized in the content of active ingredients, as innovative functional traceable ingredients, even without having to go through specific extraction and concentration technologies, and can be used as highly bioavailable antioxidants for human well-being, for a healthy and correct diet and for the prevention of diseases related to aging.

The integrated process according to the invention is divided into the following three sub-lines:
1. production of extra virgin olive oil (EVO) with a high content of polyphenols, compliant with the EFSA health specifications for olive oils eligible to protect the cardiovascular system from oxidative stress;
2. transformation of stoned virgin olive pomace into a lyophilized (freeze-dried) and micronized product with high nutraceutical value, mainly for food or nutraceutical uses;
3. transformation of leaves and apical twigs resulting from the defoliation of olives, or even from pruning, into a dried and micronized product, standardized in content of total polyphenols, and in particular of oleuropein, mainly for food, nutraceutical or cosmetic uses.

In the context of the studies connected with the present invention it has been observed that the recovery of most of the by-products of the oil industry can be carried out with economically advantageous results, as well as environmentally sustainable, if the transformation of these by-products into reusable materials takes place at the same oil plant, with innovative techniques that guarantee a high quality of the products obtained in terms of the antioxidant content.

For this reason, in a two-phase continuous pressing process, the presence of two parallel pressing lines, which can work alternatively or both, one of which works with previously stoned olives, has been foreseen, which produce, after mechanical extraction of the oil, a wet pomace without a stone. This stoned de-oiled olive paste is directly conveyed to a specially designed freeze-drying equipment, which transforms it into a solid material that has kept unchanged all the organic active ingredients initially present in the virgin pomace. Freeze-drying, also known as lyophilisation or cryodesiccation, by means of which the water contained in biological substances or matrices solidifies as a result of a sudden cooling and is eliminated by sublimation under vacuum, it is in fact used for the conservation of organic matrices also in the food sector, but it does not appear to have ever been applied for the recovery of virgin olive pomace.

Also according to the invention, it has been envisaged that the leaves and apical twigs of the olive tree, which, as is known, are rich in polyphenolic active ingredients that already have a wide market in the nutraceutical and cosmetic field, are transferred from the defoliation phase that precedes washing of the olives for pressing (possibly together with other leaves deriving from the pruning of the olive trees) in a controlled drying chamber in which they are dehydrated with calibrated temperatures and times, and in controlled gaseous composition atmospheres. The drying chamber of the plant according to the invention is equipped with a humidity extractor and sanitizing LED-UV lamps.

Each of the two solid products obtained from the two previous operations can be reduced into a fine powder by means of a micronizer, packaged, sanitized for passage in a sanitization tunnel and placed on the market.

The main process of the production platform according to the present invention, that is to say the extraction of EVO oil, in order to achieve the objective of a high content of antioxidant polyphenolic compounds, makes use of a non-destructive monitoring of the state of ripening of olives and their content in polyphenols and minor polar compounds. This monitoring is based on the innovative use of a multiparametric fluorescence sensor capable of evaluating the content of chlorophyll and polyphenols on the surface of a plant material. This sensor, called Multiplex (US patent US 8476603 B2, marketed by the French company Force-A) is a portable battery-operated instrument, which was originally designed and used for the field evaluation of red pigments and polyphenol content in grapes.

According to the present invention, the Multiplex has been adapted to a control function of the olives entering the pressing, housing it in a fixed position after the first stages of defoliation, washing and drying. In this position the sensor has the purpose of "scanning" the olives entering the process in order to acquire data on their state of ripeness. The acquired data can then be used to compare them with a database of similar data already acquired, in order to be able to determine on the basis of the previous ones the most suitable operating conditions for extracting oil from olives with similar characteristics detected.

### Brief description of the drawings

The specific features of the invention, as well as its advantages, will be more evident with reference to the attached figures, in which:
**Figure 1** shows a schematic and symbolic representation of the integrated process for the production of high-quality EVO oil and micronized by-products rich in polyphenols according to the present invention, of products obtained therefrom and relative uses;
**Figure 2** is a schematic flow chart illustrating a specific embodiment of the integrated process according to the invention.

### Detailed description of the invention

Therefore, the specific object of the present invention is an integrated process for the eco-sustainable production of extra virgin olive oil and solid by-products rich in polyphenols, wherein the following operations are carried out:
A) defoliation, twig removal and washing of the olives entering the process, comprising scanning said olives with an optical sensor, which determines the degree of ripeness, the data collected by said sensor being employed to compare them with a database of similar data already acquired and determine, on the basis of the previous data, the most suitable operation conditions for extracting oil from olives with similar characteristics, by means of a specific software provided in the subsequent extraction operation;
B) extraction of extra virgin olive oil (EVO) from said olives by mechanical means according to the continuous two-phase method, which in turn includes two production processes in parallel:
   the first one operating on olives with stone by means of ordinary cold extraction, and
   the second one operating by pressing and machine pitting the olives at the same time, with the production, from said second process, of a residue consisting of a stoned de-oiled olive paste,
   the EVO oil obtained from said second production process being cut in predetermined percentages with said EVO oil from said production processoperating on olives with stone;
C) a treatment section of said stoned de-oiled olive paste directly following said second production line, comprising a freeze-drying operation of said stoned de-oiled olive paste, with production of a solid lyophilisate containing polyphenols and of water, which is recirculated to said section A) for defoliation and washing of the olives; and
D) a treatment of olive leaves and apical twigs resulting from said section A) of defoliation and washing of the olives, and any other leaves resulting from olive tree pruning, including a drying operation in the drying chamber, with the production of a dried, containing oleuropein leaf product and of water, which is recirculated to said section A) for defoliation and washing of the olives.

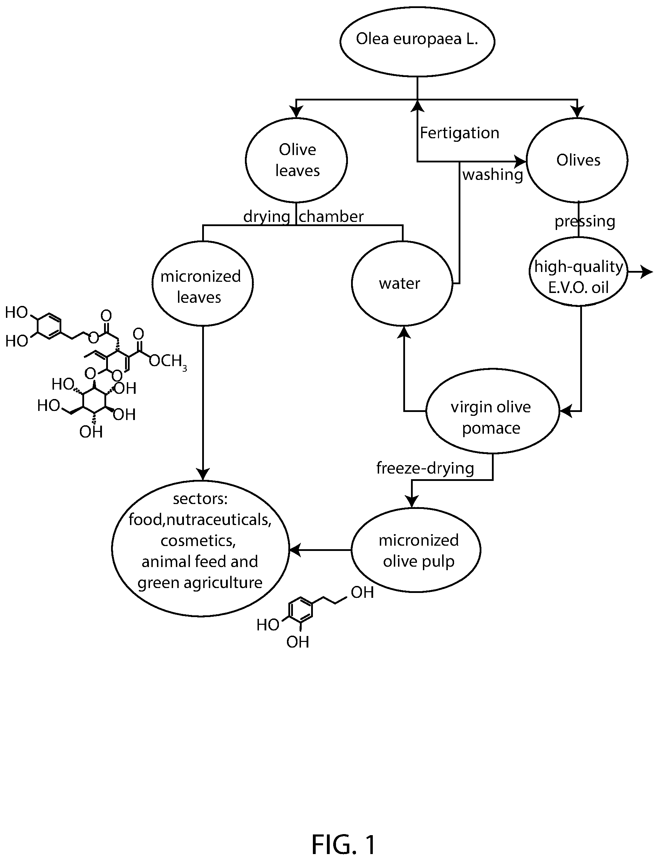

The salient features of the integrated process proposed for the production of high-quality EVO oil and for the immediate recovery of its by-products rich in polyphenols, according to the present invention, are summarized schematically in the attached **Figure 1****,** together with the indication of the products obtained from it and the sectors in which these products find their place.

Preferably, the aforementioned EVO oil extraction section includes the following preliminary operations:
a. defoliation, twig removal and stone removal of the olives fed to the process;
b. washing, rinsing and drying the olives resulting from the previous operation;
c. scanning of the olives resulting from the previous operation with a multiparametric fluorescence optical sensor, for the non-destructive measurement of the state of ripeness of the olives by determining their chlorophyll and polyphenol content.

As already noted, an instrument that meets these requirements is the Multiplex (Force-A), which in its original version is a portable fluorescence optical sensor, powered by batteries and normally usable in field, directly on the plant. The sensor comprises four LED excitation sources with spectral bands in UV-A (375 nm), blue (460 nm), green (515 nm) and red (637 nm) and three channels of photodiode detectors in the blue spectral regions - green, red and near infrared. The choice of sensor excitation sources is linked to the absorption wavelengths of the main substances of interest, such as anthocyanosides (520 nm), pigments of red or olive varieties, flavonols (350 nm). The excitation source at 375 nm, in fact, overlaps with the absorption of luteolin and apigenin, flavones or yellow pigments with an antioxidant character of the fruit, while the sources in blue and green overlap with the absorption of anthocyanosides.

According to the invention, the possible use of the instrument or of specific LEDs is also envisaged also inside the aforementioned leaf drying chamber, in order to be able to acquire specific data of functional quality and presence of active ingredients in the leaves to evaluate, during the drying phase, the content in total chlorophylls, total flavonoids and secoiridoic derivatives containing ortho-phenols. Therefore, acquisitions of chlorophyll index, flavonoid index and simple phenol index are foreseen, both on the raw material and on the post-drying products and on the post-treatment freeze-drying products obtained from said stoned de-oiled olive paste.

The additional features of the integrated process for the eco-sustainable production of high-quality EVO oil and micronized by-products rich in polyphenols according to the invention are defined in subsequent claims 3-12.

### EXAMPLE

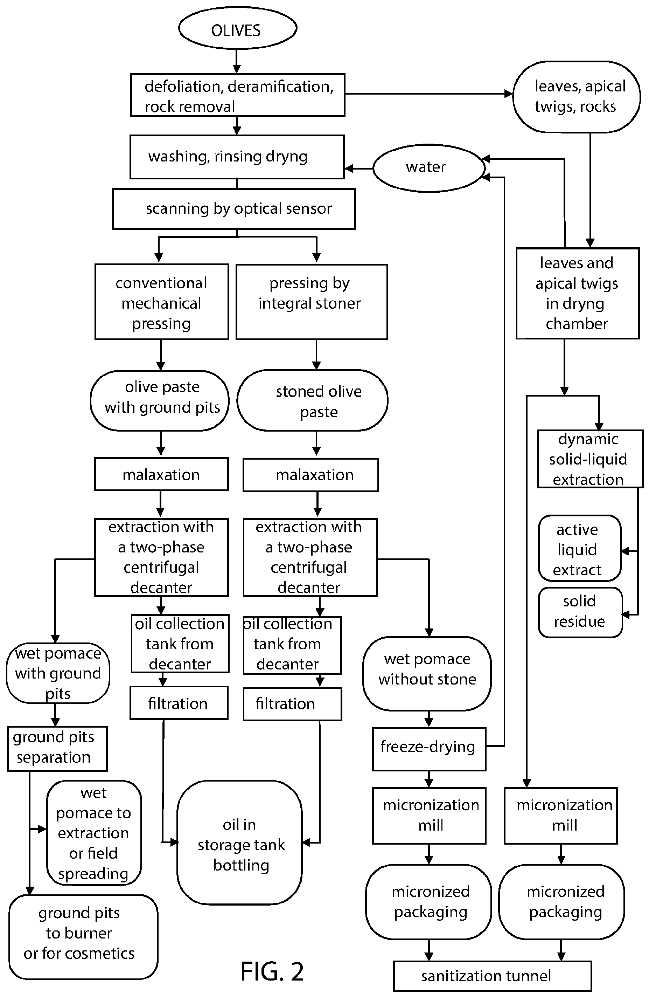

A specific embodiment of the integrated process according to the present invention is described in the following by way of mere example but not limiting, together with the results of the experiments carried out on the products obtained therefrom. The same embodiment is illustrated in the block diagram presented in **Figure 2****.**

### • Production of organic olives and biodynamic olives also through the use of green agriculture formulas from circular economy

Field treatment is envisaged with green agricultural products admitted in the biological and biodynamic synergistic treatment, products that can also be obtained from the extracts and micronized products that can be obtained from the process phases described below. These products can be mixed with natural antimicrobials, in particular with compounds belonging to the class of hydrolysable tannins, or with natural condensates or volatile aromatic molecules obtained from extraction processes in a current of steam, such as hydro-essences and essential oils.

### Features of oil mill

### • Defoliation, reception of the olives and aspiration of the leaves

The defoliation, twig removal, stone removal operation is carried out with an industrial defoliator, for example a floating compressed air industrial defoliator (Mercury).

### • Washing, rinsing, drying

Subsequently, the olives are washed with water in a recirculating washing machine for soft washing with the hydro bubble method, rinsed with disposable clean water, and dried with air blades.

According to the process of the invention, the water used for the washing phase of the olives is at least in part recovered from the subsequent phases of dehydration of the leaves and freeze-drying (lyophilization) of the stoned and de-oiled olive paste obtained from the wet pomace.

### • Non-destructive monitoring of the state of ripeness and of the content of polyphenols and minor polar compounds in the olives to be subjected to pressing.

According to the invention, the non-destructive monitoring of the ripening state of the olives and their content in polyphenols and minor polar compounds is envisaged using the Multiplex optical sensor as described above.

By placing the fluorometer in a fixed position on the inclined plane of the pressing to monitor the incoming olives, and connecting it with a computerized data acquisition and processing system, it is possible to create a database, customized by olive cultivar, relating to the content in red pigments, yellow pigments, secoiridoids (with particular reference to the oleuropein content) and chlorophyll of the treated olives. These parameters allow to have objective information on the degree of ripeness of the fruit and on the specific content of minor active compounds.

Downstream of the scanning with the Multiplex, olives enter one of two parallel extraction lines, which can be processed alternately or simultaneously, and one of which treat olives with stone (traditional pressing) and the other stoned olives .

### • Pressing

The pressing (continuous two-phase system) can be carried out in parallel with one of the following two methods:
a. Traditional cold pressing with crusher (Mori-TEM) with speed control and variable speed and control of the heat exchange, between the shell and the base, by means of water conveyed by a thermal generator, which allows to heat or cool if necessary.
b. Mechanical crushing carried out by means of an integral pitting machine (Mori-TEM), with the possible use of thermal control as required for traditional pressing (a) by making a bypass.

The olive paste obtained from each of the two pressings, in one case with ground pits and without pits in the other, then passes to the malaxation phase.

### • Malaxation

Each of the malaxators, which in the specific case are four in number, deals exclusively with one of the two olive pastes. It should be noted that each malaxator can be used for another batch of olive paste only after careful (automatic) washing of the machine with disposable water. Each malaxator is equipped with a vacuum pump, which can be equipped with gas generators (nitrogen, CO₂ and ozone).

All the malaxators have safety valves and are equipped with specific software for monitoring the whole process, thanks to which it is possible to optimize customized process methods according to the cultivar and/or the degree of ripeness of the fruit, starting from a data collection database of previous productions. All this turns out to be extremely useful for the continuous process.

The system is also enhanced by the possible remote management through the additional use of cameras.

### • Separation of EVO oil without the need for a vertical centrifuge

For each of the two production lines of the crusher, a decanter of 1200 kg/h is used with a capacity between 900-1000 kg/h without adding water, with the possibility of managing the speed of revolutions between the auger and the drum in an interconnected way through the use of a pulley.

This allows to obtain EVO oil with different extractive characteristics, and therefore with different content in minor polar antioxidant compounds, as well as different presence of pectin in the olive paste. The latter, which for example, in the phase of high maturation of the raw material, absorb oil.

### • Collection and filtration

For each of the two extraction lines there is a direct passage from the decanter to the collection tank with filter sheets (with different granulometry), connected to a pump with inverter which regulates the flow rate of the filter.

In this phase it is possible to use on-line control sensors at the output, such as a turbidimeter that allows the selection of the most suitable filter to exclude the risk of sedimentation.

### • Pre-bottling collection and storage tanks

The oil from traditional pressing and the oil from stoned olives are then sent to different tanks positioned in an air-conditioned environment and equipped with an internal temperature control sensor, and with the use of inert gas (e.g. argon) for optimal conservation of quality characteristics of the oil.

### • EVO oil bottling line

Bottling takes place after any cutting between the two types of oil, in order to optimally calibrate the organoleptic characteristics, structure and shelf life of the resulting blend.

The bottling line is equipped with an inert gas system, preferably argon, to keep the quality of the oil unchanged for as long as possible.

### Recovery of the stoned wet virgin pomace from a two-phase process

### • Spray freeze-drying to obtain active powders from stoned de-oiled olive paste

The integrated process according to the invention provides for the sending of virgin pomace obtained from stoned olives directly to a freeze-drying (lyophilizing) spray system, for the production of active powders also functional as stoned de-oiled olive paste.

Out of the decanter, the stoned de-oiled olive paste is pumped into a system where the presence of liquid nitrogen, or of a refrigerator-reference group, allows the pasta to be frozen, which when nebulized, vacuum-packed, undergoes a fast process of dehydration in soft conditions for the maintenance of the content in active compounds, with particular reference to hydroxytyrosol, a primary molecule for the antioxidant characteristics in products and for the protection of human health.

Cold vacuum freeze-drying with a capacity of 200 kg/hour of product at 75% of water. The product obtained will have a water content <1% with a maximum treatment temperature of 30°C.

The plant consists of:
a) Instant product cooler
b) Microwave vacuum freeze dryer

### a) Instant cooler for frozen liquid product

The product to be treated is pumped at high pressure into a heat exchange chamber where a rotating nebulizer nebulizes it in drops having sizes of the order of 200 microns.

The machinery consists of:
▪ 1000 litre storage tank complete with mixer used to homogenize the product;
▪ high pressure pump with a capacity of 400 litres/hour, maximum pressure 16 bar;
▪ spray chamber made of stainless steel, entirely coated with a thickness of 100 mm in high-density polyurethane foam;
▪ air flow separation cycle of ice particles, equipped with a guillotine valve used for unloading onto the conveyor screw;
▪ rotating nebulizer at 15000 rpm;
▪ producer of cold air at -10°C with air-cooled refrigeration unit, thermal power 100 kW;
▪ blower with a maximum flow rate of 5000 cubic meters/hour controlled by an inverter;
▪ heat exchanger;
▪ electrical command and control panel.

Liquid nitrogen can be used instead of the cooling system by means of a refrigeration unit.

### b) Continuous vacuum freeze dryer

The machine, with a capacity of 200 kg/h of product to be treated, is made up of:
▪ tube heat exchanger equipped with product feed screw,
▪ star valve located at the outlet of the exchanger used to guarantee the maintenance of the vacuum;
▪ vacuum intake tube;
▪ condenser used to condense vapours, complete with sprayers to inject the product and prevent the exchanger tubes from freezing;
▪ liquid ring vacuum pump;
▪ refrigeration compressor with thermal 100 kW at -10°C;
▪ electrical operational and control panel.

The condensation water is 150 kg/hour with a natural composition from olive, and is reusable both in the washing processes of the raw material and in fertigation.

### Olive leaf treatment line

The leaf collection system provides, as described, the defoliation operation carried out in the mill, before pressing, and the presence of a basket for collecting the leaves within the crushing area itself or in a neighboring covered area.

### • Drying of the olive leaves in a controlled drying chamber

This phase of the process involves the drying of olive leaves, harvested at different balsamic times. The operation has a high added value due to the presence of antioxidant components, with particular reference to oleuropein.

Below are the specifications of the drying chamber according to a preferred embodiment of the invention.
1) Monitoring of the active ingredients of the fresh raw material, as input data.
2) Customization of the laying of the material to be dried in controlled and monitored thicknesses in trays/baskets of free material, equipped with sensors for thermal control, humidity and movement, if necessary, of the container itself, all housed in mobile pallets.
3) Dehydration in a controlled room equipped with a humidity extractor at controlled speed and suction capacity, presence of insulated walls with high prevalence flat fans and equipped with computer-controlled inverters for the control of flows and air circulation. Presence of both directional and diffusion sanitizing UV LEDs, of UV sanitizing and IR heating lamps (if necessary), and of controlled inputs for gases such as nitrogen, ozone, carbon dioxide and argon to be customized for modification and control of the process atmosphere. The chamber is equipped with temperature, humidity and gas composition control sensors.
4) Equipped with nitrogen generator and ozone generator and housing of argon and carbon dioxide cylinders. All gases can be used both in the controlled drying and sanitization phases and as conditioning and sanitization of the environment and of the pipes, with management phases controlled by dedicated software, with personalization and storage of parameters and ramps as well of temperature, gas flows and humidity suction associated with activations with individual ramps and times of use of LEDs and UV lamps.
5) Interior of the chamber in fully reflective food-compatible material and, depending on the required dimensions, the chamber itself can be equipped with an internal partition between the pallets, of minimum thickness, made of reflective material equipped with UV light LEDs widespread.
6) Supply of condensate recovery container for food use, as a collection of micellar water, free from salts, usable both in the cosmetic sector and as a raw material in the design and manufacture of products for sanitization, including industrial.
7) The chamber can be mobile or semi-mobile by means of use of a bed using a thickness of 15 cm from the ground or mobile trolley on wheels.
8) Possible extra equipment of offline systems, control of product humidity and residual water activity in order to ensure the legal parameters of drying or dehydration required for each vegetable matrix.

The leaves can then be micronized as described below, for the production of standardized powders in oleuropein content.

### • Micronization to obtain active powders from olive leaf and dehydrated olive paste

Both for the stoned de-oiled and freeze-dried olive paste and for the dried leaves as described above, reduction into a fine powder is envisaged by means of a mechanical blade and/or mechanical ball micronizer. The micronizer is made of steel for food use, and allows to obtain powders with differentiated grain size, with temperature and humidity control and, according to the equipment of other areas of the plant, it has the possibility of mechanical control and of inertizable blanket.

### • Active powder packaging line

The process of the invention provides for a post-micronization packaging system in the absence of air and sanitization, according to regulations, of the packaged product by means of a tunnel with UV-C LEDs.

In particular, both for the micronized from olive leaves previously sanitized in the drying chamber, and for the micronized products obtained by freeze-drying stoned de-oiled olive paste, it is envisaged to be packaged in polymeric bags permeable to UV-C radiation with controlled thickness, in order to correctly eliminate internal oxygen and allow the permeation of sanitizing radiations on at least 95% of the surface of the packaged powders.

The designed sanitizing tunnel is also designed for packaging in an inert atmosphere, and provides for irradiation of the entire packaging surface, with the presence of a UV-C irradiation time control sensor.

### Alternative treatment of dried olive leaves

### • Dynamic solid-liquid extraction of dried leaves

As an alternative to micronization for the marketing of olive leaf powder in solid form, to be used for example for antioxidant herbal teas with a high oleuropein content, the leaves thus dried can also be subjected to dynamic liquid-solid extraction, in order to obtain standardized liquid solutions in oleuropein content.

The dynamic liquid-solid extraction is governed by the principle according to which the generation, with a suitable solvent, of a negative pressure gradient between the inside and the outside of a solid that contains extractable material, followed by a sudden restoration of the conditions of initial equilibrium, induces the forced extraction of the compounds not chemically bound to the main structure of which the solid is constituted. On the basis of this principle, pressure and depression cycles are applied to the extracting liquid which is in contact with the vegetable solid matrix, from which the active ingredients are extracted with extreme efficiency (Naviglio Extractor).

From the described operation, a liquid extract rich in oleuropein and a solid residue are obtained which can be used for field spreading as a bio-fertilizer.

### Treatment of wet virgin pomace with ground pits from a two-phase process

The portion of wet pomace coming from the traditional extraction line, which contains ground pits, can be subjected to separation to recover the ground pits.

### • Separation of the ground pits for energy purposes and cosmetic uses

In addition to the use of the ground pits for energy purposes, it is possible to treat the latter in a micronizer in order to obtain powders that can be used both in cosmetics, such as powders for scrubs, and for the formulation of green agricultural products.

Finally, the fraction of wet pomace that remains from the separation of the ground pits can be delivered for the production of biogas, or used for field spreading, or even be sent to pomace oil extraction.

### Study of the EVO oil produced

Two different types of EVO oil, produced according to the technologies of the present invention and referred to here as Sample A and Sample B, have been subjected to analysis to verify their quality in terms of controlled acidity, low number of peroxides and content in polyphenolic compounds complying with the indications of EFSA (EU No. 432/2012).

The qualitative-quantitative analysis with HPLC-DAD-MS (high performance liquid chromatography coupled with a diode array detector and mass spectrometry) was carried out at three control points:
1. at bottling;
2. after 6 months from bottling;
3. after 18 months from bottling.

### 1. Control point at bottling

| *Compounds* | *Sample A mg*/*L* | *Sample B mg*/*L* |
|---|---|---|
| Hydroxytyrosol | 9.73 | 10.26 |
| Tyrosol | 7.98 | 6.97 |
| Elenolic acid derivatives | 9.28 | 7.13 |
| Elenolic acid | 55.06 | 44.62 |
| 10-hydroxyoleocanthal | 149.7 | 209.49 |
| Oleocanthal | 164.5 | 143.57 |
| Summation of secoiridoid derivatives | 171.01 | 153.25 |
| Lignans | 85.91 | 79.73 |
| Oleuropein aglycone | 170.37 | 141.24 |
| Total minor polar compounds (MPCs) | 823.53 | 796.26 |
| MPC excluding elenolic acid and derivatives | 759.2 | 744.51 |

| Determination of the number of active meq O₂ peroxides/kg | | |
|---|---|---|
| *Sample A* | 11.34 | |
| *Sample B* | 11.84 | |

| Analysis of free acidity expressed as oleic acid (%) | |
|---|---|
| *Sample A* | 0.09 |
| *Sample B* | 0.11 |

| Determination of the number of polyphenols (mg/kg Tyrosol) | |
|---|---|
| *Sample A* | 769 |
| *Sample B* | 879 |

### 2. Control point after 6 months

| *Compounds* | *Sample A mg*/*L* | *Sample B mg*/*L* |
|---|---|---|
| Hydroxytyrosol | 18.43 | 16.46 |
| Tyrosol | 12.61 | 10.07 |
| Elenolic acid derivatives | 7.73 | 9.14 |
| Elenolic acid | 66.86 | 59.73 |
| 10-hydroxyoleocanthal | 119.22 | 211.47 |
| Oleocanthal | 126.42 | 118.55 |
| Summation of secoiridoid derivatives | 158.06 | 87.28 |
| Lignans | 69.91 | 65.44 |
| Oleuropein aglycone | 171.30 | 137.08 |
| Total minor polar compounds (MPCs) | 750.53 | 715.23 |
| MPC excluding elenolic acid and derivatives | 675.95 | 646.36 |

| Determination of the number of active meq O₂ peroxides/kg | |
|---|---|
| *Sample A* | 11.2 |
| *Sample B* | 12.5 |

| Analysis of free acidity expressed as oleic acid (%) | |
|---|---|
| *Sample A* | 0.29 |
| *Sample B* | 0.42 |

| Determination of the number of polyphenols (mg/kg Tyrosol) | |
|---|---|
| *Sample A* | 517 |
| *Sample B* | 574 |

### 3. Control point after 18 months

| *Compounds* | *Sample A mg*/*L* | *Sample B mg*/*L* |
|---|---|---|
| Hydroxytyrosol | 0.41 | 0.78 |
| Tyrosol | 0.06 | 0.38 |
| Elenolic acid derivatives | 11.39 | 13.23 |
| Elenolic acid | 65.01 | 62.41 |
| 10-hydroxyoleocanthal | 70.56 | 84.28 |
| Oleocanthal | 68.68 | 76.55 |
| Summation of secoiridoid derivatives | 33.41 | 35.71 |
| Lignans | 24.56 | 28.27 |
| Oleuropein aglycone | 98.22 | 83.01 |
| Total minor polar compounds (MPCs) | 372.31 | 384.62 |
| MPC excluding elenolic acid and derivatives | 295.91 | 308.98 |

| Determination of the number of active meq O₂ peroxides/kg | |
|---|---|
| *Sample A* | 12.97 |
| *Sample B* | 11.17 |

| Analysis of free acidity expressed as oleic acid (%) | |
|---|---|
| *Sample A* | 0.72 |
| *Sample B* | 0.59 |

| Determination of the number of polyphenols(mg/kg Tyrosol) | |
|---|---|
| *Sample A* | 376 |
| *Sample B* | 394 |

The limits imposed in order to consider EVO oil as eligible for a health claim according to EU Regulation No. 432/2012 are, as is known, 5 mg of hydroxytyrosol and its derivatives per 20 g of oil. In total, therefore, 250 mg of hydroxytyrosol and derivatives per kg of EVO oil.

The total of compounds whose content in the oil must be counted for this purpose is the sum of the active molecules such as hydroxytyrosol, 10-hydroxyoleocanthal and oleuropein aglycone.

As can be seen, the two samples A and B given as an example largely comply with the request to allow an intake of at least 5 mg of hydroxytyrosol and derivatives in 20 of oil, necessary to be able to consider them beneficial for cardiovascular prevention.

In addition, the results obtained from the characterization of two other samples of EVO oil obtained by implementing the integrated process of this invention and the related considerations for compliance with the requirements of EFSA are reported. The samples are referred to here as Sample C and Sample D.

HPLC-DAD-MS characterization analysis of the two EVO oils Sample C and Sample D

| *Compounds* | *Sample C mg*/*L* | *Sample D mg*/*L* |
|---|---|---|
| Hydroxytyrosol | 9.29 | 7.90 |
| Tyrosol | 6.23 | 6.13 |
| Elenolic acid derivatives | 36.90 | 30.40 |
| Elenolic acid | 80.42 | 70.74 |
| 10- hydroxyoleocanthal | 193.17 | 162.82 |
| Oleocanthal | 31.22 | 49.53 |
| Summation of secoiridoid derivatives | 34.35 | 23.39 |
| Lignans | 152.24 | 166.05 |
| Oleuropein aglycone | 112.99 | 121.01 |
| MPC excluding elenolic acid and derivatives | 656.81 | 637.96 |
| MPC excluding elenolic acid and derivatives | 539.48 | 536.82 |

### Analysis of free acidity expressed as oleic acid (%)

| | |
|---|---|
| *Sample C* | 0.21 |
| *Sample D* | 0.16 |

### Determination of the number of active meq O₂ peroxides/kg

| | |
|---|---|
| *Sample C* | 7.79 |
| *Sample D* | 6.98 |

Considering also in this case the compounds that contribute to the count of beneficial active ingredients according to EFSA, namely hydroxytyrosol, tyrosol, 10-hydroxyoleocanthal and oleuropein aglycone, it appears that for the EVO oil of Sample C the contents of these compounds correspond to a total of 315.45 mg/L, equal to 342.88 ppm, which correspond to 6.8 mg compared to the 5 mg required.

For EVO oil Sample D, the levels of these compounds correspond to a total of 291.76 mg / L, equal to 317.13 ppm, corresponding to 6.3 mg compared to the 5 mg required.

### Study of the functional quality of olive leaves and stoned de-oiled olive paste.

Production of standardized micronized products in **oleuropein** from olive leaves

| *Compounds* | *Micronized olive leaves cv Seggianese in pruning (mg*/*g)* | *Micronized olive leaves cv Seggianese in pressing (mg*/*g)* |
|---|---|---|
| Hydroxytyrosol glucoside | 1.62 ± 0.11 | 1.31 ± 0.05 |
| Hydroxytyrosol | 1.02 ± 0.04 | Nd |
| Elenolic acid diglucoside | 10.33 ± 0.61 | 9.26 ± 0.41 |
| Derivative caffeic acid | 2.21 ± 0.09 | Nd |
| Verbascoside | 5.28 ± 0.24 | 3.01 ± 0.12 |
| Luteolin 7-O-glucoside | 3.60 ± 0.14 | 1.83 ± 0.07 |
| Oleuropein | 217.4 ± 15.8 | 110.16 ± 9.13 |
| Oleuropein aglycone | 27.84 ± 1.13 | 2.51 ± 0.10 |
| Total polyphenols | 269.30 ± 18.16 | 128.08 ± 9.88 |

### Production of standardized micronized products in hydroxytyrosol from olive paste

| *Compounds* | *Olive paste (mg*/*kg)* | *Olive paste de-oiled and dried (mg*/*kg)* |
|---|---|---|
| Hydroxytyrosol | 6917.0 ± 269 | 1266.4 ± 48.1 |
| Tyrosol | 3744.1 ± 146 | 415.9 ± 15.8 |
| Verbascoside | 5830 ± 227 | 183.6 ± 7.16 |
| Total polyphenols | 16491.1 ± 642 | 1865.9 ± 71.06 |

As previously described in relation to the use of Multiplex technology, the micronized from olive leaf and stoned and de-oiled olive paste were also characterized using this instrumentation. In particular, it was possible to evaluate the content in total chlorophylls, total flavonoids and secoiridoid derivatives containing ortho-phenols, in order to obtain innovative parameters with a non-destructive method to be included in the product data sheet.

Both the micronized from leaves and the stoned and de-oiled olive paste, standardized in the content of active ingredients and fibres, can be used in the food, nutraceuticals, cosmetics, animal feed and green agriculture sectors. Their use as active ingredients, with particular regard to the food and nutraceutical sectors, allows the formulation of products that can guarantee the daily intake of portions or doses which, containing 1-5 grams of micronized olive paste and from leaf as a single ingredient or in mixture, per kg of micronized product, they guarantee an intake of hydroxytyrosol and derivatives of 5 mg/day as prescribed in the EFSA health specifications indicated above.

The present invention has been described with reference to some of its specific embodiments, but it is to be understood that variations or modifications may be made to it by those skilled in the art without thereby departing from the scope defined by the claims.

## Claims

1. An integrated process for the eco-sustainable production of extra virgin olive oil and solid by-products rich in polyphenols, wherein the following operations are carried out:
A) defoliation, twig removal and washing of the olives entering the process, comprising scanning said olives with an optical sensor, which determines the degree of ripeness, the data collected by said sensor being employed to compare them with a database of similar data already acquired and determine on the basis of the previous data the most suitable operation conditions for extracting oil from olives with similar characteristics, by means of a specific software provided in the subsequent extraction operation;
B) extraction from said olives of extra virgin olive oil (EVO) by mechanical means according to the continuous two-phase method, which in turn includes two production processes being carried out in parallel:
the first one operating on olives with stone by means of ordinary cold extraction, and
the second one operating by pressing and machine pitting the olives at the same time, with the production, from said second process, of a residue consisting of a stoned de-oiled olive paste,
the EVO oil obtained from said second production process being cut in predetermined percentages with the EVO oil obtained from said first production process operating on olives with stone;
C) treatment of said stoned de-oiled olive paste directly following said second production process, comprising a freeze-drying operation of said stoned de-oiled olive paste, with production of a solid lyophilisate containing polyphenols and of water, which is recirculated to said operation A) of defoliation and washing of the olives; and
D) treatment of olive leaves and apical twigs resulting from said operation A) of defoliation and washing of the olives, and possibly other leaves resulting from olive tree pruning, including a drying operation in a drying chamber, with the production of a dried leaf product containing oleuropein and of water, which is recirculated to said operation A) of defoliation and washing of the olives.

2. The integrated process according claim 1, wherein said operation A) of defoliation and washing of olives comprises the following operations:
a) defoliation, twig removal and rock removal of the olives fed to the process;
b) washing, rinsing and drying the olives resulting from the previous operation;
c) scanning of the olives resulting from the previous operation by means of a multiparametric fluorescence optical sensor, to obtain a non-destructive measurement of the state of ripeness of the olives by determining their chlorophyll and polyphenol contents;
d) storing the data obtained from operation c) in a computerised system of data acquisition and processing, provided with a specific software for driving and regulating said subsequent extraction operation B).

3. The integrated process according to claims 1 o 2, wherein said second production process comprises the following operations:
e) pressing the olives under vacuum or in a controlled atmosphere, by means of an integral pitting machine, with separation of the olive paste from the stone;
f) malaxation of olive paste under vacuum or in a controlled atmosphere, and at a controlled temperature;
g) extraction of EVO oil with a two-phase centrifugal decanter, resulting in oil output on one side and de-oiled stoned olive paste output on the other;
h) direct passage of the extracted oil to a collection tank with filter;
i) collection in a storage tank of EVO oil from stoned olives, in an air-conditioned environment and with the possible use of inert gas;
j) mixing in predetermined percentages with EVO oil obtained from said first production operation operating on olives with stone;
k) bottling under inert gas blanket the oil obtained from the previous operation i) or j).

4. The integrated process according claim 3, wherein said de-oiled stoned olive paste obtained from said operation g) is directly fed to the freeze-drying operation of the treatment operation of the said de-oiled stoned olive paste.

5. The integrated process according to any one of claims 1-4, wherein said treatment operation C) of the de-oiled stoned olive paste also comprises, after said freeze-drying operation, a micronization operation of said solid lyophilisate, resulting in the production of a micronized product rich in polyphenols having antioxidant activity.

6. The integrated process according to claim 5, wherein said treatment operation C) of the de-oiled stoned olive paste also comprises, after said micronization operation, a packaging operation, followed by a final treatment in a sanitization tunnel with UV-C rays.

7. The integrated process according to any one of claims 1-6, wherein said operation D) of treatment of olive leaves and apical twigs also comprises, after said drying operation, a micronization operation of said dry leaf product, with production of a micronized rich in oleuropein with antioxidant activity.

8. The integrated process according to claim 7, wherein said operation D) of treatment of olive leaves and apical twigs also comprises, after said micronization operation, a packaging operation, followed by a final treatment in a sanitization tunnel with UV-C rays.

9. The integrated process according to claim 3, wherein the EVO oil obtained from said bottling operation k) has a content of hydroxytyrosol and derivatives thereof higher than 250 mg/kg.

10. The integrated process according to claim 5, wherein the product obtained from said operation of micronization of the solid lyophilisate of de-oiled stoned olive paste has a content of hydroxytyrosol and derivatives higher than 1500 mg/kg.

11. The integrated process according to claim 7, wherein the micronized product obtained from said operation of micronization of said dry leaf product has an oleuropein content higher than 15% by weight.

## Patentansprüche

1. Integriertes Verfahren zur ökologisch nachhaltigen Herstellung von hochwertigem extranärem Olivenöl und festen Nebenprodukten, die reich an Polyphenolen sind, wobei die folgenden Arbeitsschritte durchgeführt werden:
A) Entblättern, Entfernen der Zweige und Waschen der in den Prozess eintretenden Oliven, wobei die Oliven mit einem optischen Sensor abgetastet werden, der den Reifegrad bestimmt, wobei die von dem Sensor gesammelten Daten verwendet werden, um sie mit einer Datenbank ähnlicher, bereits erfasster Daten zu vergleichen und auf der Grundlage der früheren Daten die am besten geeigneten Betriebsbedingungen für die Extraktion von Öl aus Oliven mit ähnlichen Merkmalen zu bestimmen, und zwar mit Hilfe einer spezifischen Software, die in dem nachfolgenden Extraktionsvorgang bereitgestellt wird;
B) Extraktion von nativem Olivenöl extra (EVO) aus den Oliven mit mechanischen Mitteln nach dem kontinuierlichen Zweiphasenverfahren, das seinerseits zwei parallel ablaufende Produktionsprozesse umfasst:
der erste bearbeitet Oliven mit Stein durch gewöhnliche Kaltextraktion, und
das zweite Verfahren, bei dem die Oliven gleichzeitig gepresst und maschinell entkernt werden, wobei aus dem zweiten Verfahren ein Rückstand entsteht, der aus einer entkernten und entölten Olivenpaste besteht,
das EVO-Öl, das aus dem zweiten Herstellungsverfahren gewonnen wird, wird in vorbestimmten Prozentsätzen mit dem EVO-Öl, das aus dem ersten Herstellungsverfahren gewonnen wird, das mit Oliven mit Stein arbeitet, gemischt;
C) Behandlung der entsteinten, entölten Olivenpaste direkt im Anschluss an das zweite Herstellungsverfahren, die eine Gefriertrocknung der entsteinten, entölten Olivenpaste umfasst, wobei ein festes, Polyphenole und Wasser enthaltendes Lyophilisat erzeugt wird, das in den Vorgang A) des Entblätterns und Waschens der Oliven zurückgeführt wird; und
D) Behandlung von Olivenblättern und spitzen Zweigen, die aus dem Vorgang A) des Entblätterns und Waschens der Oliven stammen, und möglicherweise anderen Blättern, die aus dem Beschneiden von Olivenbäumen stammen, einschließlich eines Trocknungsvorgangs in einer Trockenkammer, mit der Herstellung eines getrockneten Blattprodukts, das Oleuropein und Wasser enthält, das zu dem Vorgang A) des Entblätterns und Waschens der Oliven zurückgeführt wird.

2. Integriertes Verfahren nach Anspruch 1, wobei der Vorgang A) des Entblätterns und Waschens von Oliven die folgenden Arbeitsschritte umfasst:
a) Entblätterung, Entfernung von Zweigen und Steinen von den Oliven, die dem Prozess zugeführt werden;
b) Waschen, Spülen und Trocknen der Oliven aus dem vorangegangenen Arbeitsgang;
c) Abtasten der aus dem vorangegangenen Arbeitsgang hervorgegangenen Oliven mit Hilfe eines optischen Multiparameter-Fluoreszenzsensors, um eine zerstörungsfreie Messung des Reifegrads der Oliven durch Bestimmung ihres Chlorophyll- und Polyphenolgehalts zu erhalten;
d) Speicherung der aus dem Vorgang c) erhaltenen Daten in einem computergestützten System zur Datenerfassung und -verarbeitung, das mit einer spezifischen Software zur Steuerung und Regelung des nachfolgenden Extraktionsvorgangs B) ausgestattet ist.

3. Integriertes Verfahren nach einem der Ansprüche 1 oder 2, wobei das zweite Herstellungsverfahren die folgenden Schritte umfasst:
e) Pressen der Oliven unter Vakuum oder kontrollierter Atmosphäre mit Hilfe einer integrierten Entkernungsmaschine, wobei der Olivenbrei vom Stein getrennt wird;
f) Malaxation der Olivenpaste unter Vakuum oder in kontrollierter Atmosphäre und bei kontrollierter Temperatur;
g) Extraktion von EVO-Öl mit einem Zweiphasen-Zentrifugal-Dekanter, der auf der einen Seite Öl und auf der anderen Seite entölte, entsteinte Olivenpaste ausgibt;
h) direkte Weiterleitung des abgesaugten Öls in einen Auffangbehälter mit Filter;
i) Sammlung von EVO-Öl aus entkernten Oliven in einem Lagertank, in einer klimatisierten Umgebung und möglicherweise unter Verwendung von Inertgas;
j) Mischen in vorbestimmten Prozentsätzen mit EVO-Öl, das aus dem ersten Produktionsvorgang an Oliven mit Stein gewonnen wurde;
k) Abfüllung des aus dem vorangegangenen Vorgang i) oder j) gewonnenen Öls unter Inertgasabdeckung.

4. Integriertes Verfahren nach Anspruch 3, wobei die entölte entsteinte Olivenpaste, die aus dem Arbeitsgang g) erhalten wird, direkt dem Gefriertrocknungsvorgang des Behandlungsvorgangs der entölten entsteinten Olivenpaste zugeführt wird.

5. Integriertes Verfahren nach einem der Ansprüche 1 bis 4, wobei der Behandlungsvorgang C) der entölten, entsteinten Olivenpaste nach dem Gefriertrocknungsvorgang auch einen Mikronisierungsvorgang des festen Lyophilisats umfasst, was zur Herstellung eines mikronisierten Produkts führt, das reich an Polyphenolen mit antioxidativer Aktivität ist.

6. Integriertes Verfahren nach Anspruch 5, wobei der Behandlungsvorgang C) der entölten, entsteinten Olivenpaste nach dem Mikronisierungsvorgang auch einen Verpackungsvorgang umfasst, gefolgt von einer Endbehandlung in einem Desinfektionstunnel mit UV-C-Strahlen.

7. Integriertes Verfahren nach einem der Ansprüche 1 bis 6, wobei der Arbeitsgang D) der Behandlung von Olivenblättern und -zweigen nach dem Trocknungsvorgang auch einen Mikronisierungsvorgang des trockenen Blattprodukts umfasst, bei dem ein mikronisiertes Produkt hergestellt wird, das reich an Oleuropein mit antioxidativer Aktivität ist.

8. Integriertes Verfahren nach Anspruch 7, wobei der Arbeitsgang D) der Behandlung von Olivenblättern und Olivenzweigen nach dem Mikronisierungsvorgang auch einen Verpackungsvorgang umfasst, gefolgt von einer Endbehandlung in einem Desinfektionstunnel mit UV-C-Strahlen.

9. Integriertes Verfahren nach Anspruch 3, wobei das aus dem Abfüllvorgang k) erhaltene EVO-Öl einen Gehalt an Hydroxytyrosol und dessen Derivaten von mehr als 250 mg/kg aufweist.

10. Integriertes Verfahren nach Anspruch 5, wobei das aus dem Vorgang der Mikronisierung des festen Lyophilisats der entölten entsteinten Olivenpaste erhaltene Produkt einen Gehalt an Hydroxytyrosol und Derivaten von mehr als 1500 mg/kg aufweist.

11. Integriertes Verfahren nach Anspruch 7, wobei das mikronisierte Produkt, das aus dem Vorgang der Mikronisierung des trockenen Blattprodukts erhalten wird, einen Oleuropeingehalt von mehr als 15 Gew.-% aufweist.

## Revendications

1. Processus intégré de production écolo-durable d'huile d'olive vierge extra et de sous-produits solides riches en polyphénols, dans lequel les opérations suivantes sont effectuées:
A) défoliation, élimination des rameaux et lavage des olives entrant dans le processus, comprenant le balayage desdites olives avec un capteur optique qui détermine le degré de maturité, les données recueillies par ledit capteur étant utilisées pour les comparer à une base de données de données similaires déjà acquises et déterminer, sur la base des données précédentes, les conditions opérationnelles les plus appropriées pour extraire l'huile d'olive présentant des caractéristiques similaires, au moyen d'un logiciel spécifique fourni lors de l'opération d'extraction ultérieure;
B) l'extraction de ces olives de l'huile d'olive vierge extra (EVO) par des moyens mécaniques selon la méthode continue à deux phases, qui comprend à son tour deux processus de production menés en parallèle:
la première opérant sur des olives à noyaux par extraction ordinaire à froid, et
la seconde opérant simultanément le pressage et le dénoyautage mécanique des olives, avec la production, à partir de cette seconde opération, d'un résidu constitué par une pâte d'olive déshuilée et dénoyautée,
l'huile EVO obtenue à partir de ce deuxième processus de production est coupée en pourcentages prédéterminés avec l'huile EVO obtenue à partir de ce premier processus de production opérant sur des olives avec noyau;
C) traitement de ladite pâte d'olive dénoyautée et déshuilée directement après ledit deuxième processus de production, comprenant une opération de lyophilisation de ladite pâte d'olive dénoyautée et déshuilée, avec production d'un lyophilisat solide contenant des polyphénols et de l'eau, qui est recirculé vers ladite opération A) de défoliation et de lavage des olives; et
D) traitement des feuilles d'olivier et des rameaux apicaux résultant de ladite opération A) de défoliation et de lavage des olives, et éventuellement d'autres feuilles résultant de la taille des oliviers, comprenant une opération de séchage dans une chambre de séchage, avec production d'un produit foliaire séché contenant de l'oleuropéine et de l'eau, qui est recirculé vers ladite opération A) de défoliation et de lavage des olives.

2. Processus intégré selon la revendication 1, dans lequel l'opération A) de défoliation et de lavage des olives comprend les opérations suivantes:
a) la défoliation, l'élimination des rameaux et l'enlèvement des pierres des olives introduites dans le processus;
b) le lavage, le rinçage et le séchage des olives résultant de l'opération précédente;
c) le balayage des olives résultant de l'opération précédente au moyen d'un capteur optique multiparamétrique à fluorescence, afin d'obtenir une mesure non destructive de l'état de maturité des olives par la détermination de leur teneur en chlorophylle et en polyphénols;
d) stocker les données obtenues à partir de l'opération
c) dans un système informatisé d'acquisition et de traitement des données, doté d'un logiciel spécifique pour la conduite et la régulation de l'opération d'extraction ultérieure B).

3. Processus intégré selon les revendications 1 à 2, dans lequel le deuxième processus de production comprend les opérations suivantes:
e) le pressage des olives sous vide ou sous atmosphère contrôlée, à l'aide d'un dénoyauteur intégré, avec séparation de la pâte d'olive du noyau;
f) la malaxation de la pâte d'olive sous vide ou dans une atmosphère contrôlée et à une température contrôlée;
g) extraction de l'huile EVO à l'aide d'un décanteur centrifuge à deux phases, ce qui permet d'obtenir d'un côté l'huile et de l'autre la pâte d'olive dénoyautée et déshuilée;
h) passage direct de l'huile extraite dans un réservoir de collecte avec filtre;
i) collecte dans un réservoir de stockage de l'huile EVO provenant d'olives dénoyautées, dans un environnement climatisé et avec l'utilisation éventuelle d'un gaz inerte;
j) mélanger à l'huile EVO, dans des pourcentages prédéterminés, l'huile obtenue à partir de la première opération de production opérée sur des olives avec noyau;
k) embouteillage sous couverture de gaz inerte de l'huile obtenue à l'issue de l'opération i) ou j) précédente.

4. Processus intégré selon la revendication 3, dans lequel ladite pâte d'olive dénoyautée et déshuilée obtenue à l'issue de ladite opération g) est directement introduite dans l'opération de lyophilisation de l'opération de traitement de ladite pâte d'olive dénoyautée et déshuilée.

5. Processus intégré selon l'une des revendications 1 à 4, dans lequel ladite opération de traitement C) de la pâte d'olive dénoyautée et déshuilée comprend également, après ladite opération de lyophilisation, une opération de micronisation dudit lyophilisat solide, aboutissant à la production d'un produit micronisé riche en polyphénols ayant une activité antioxydante.

6. Processus intégré selon la revendication 5, dans lequel ladite opération de traitement C) de la pâte d'olive dénoyautée et déshuilée comprend également, après ladite opération de micronisation, une opération d'emballage, suivie d'un traitement final dans un tunnel de désinfection aux rayons UV-C.

7. Processus intégré selon l'une quelconque des revendications 1 à 6, dans lequel ladite opération D) de traitement des feuilles d'olivier et des rameaux apicaux comprend également, après ladite opération de séchage, une opération de micronisation dudit produit foliaire sec, avec production d'un micronisé riche en oleuropéine à activité antioxydante.

8. Processus intégré selon la revendication 7, dans lequel ladite opération D) de traitement des feuilles d'olivier et des rameaux apicaux comprend également, après ladite opération de micronisation, une opération d'emballage, suivie d'un traitement final dans un tunnel de désinfection aux rayons UV-C.

9. Processus intégré selon la revendication 3, dans lequel l'huile EVO obtenue à l'issue de l'opération d'embouteillage k) a une teneur en hydroxytyrosol et ses dérivés supérieure à 250 mg/kg.

10. Processus intégré selon la revendication 5, dans lequel le produit obtenu par ladite opération de micronisation du lyophilisat solide de pâte d'olive dénoyautée déshuilée a une teneur en hydroxytyrosol et dérivés supérieure à 1500 mg/kg.

11. Processus intégré selon la revendication 7, dans lequel le produit micronisé obtenu à partir de l'opération de micronisation du produit de feuilles sèches a une teneur en oleuropéine supérieure à 15 % en poids.
